# EUROPEAN PATENT SPECIFICATION

(11) **EP 4 346 704 B1**
(45) Date of publication and mention of the grant of the patent: **19.11.2025**
(21) Application number: 22731375.6
(22) Date of filing: 19.05.2022
(51) Int. Cl.: A61F 2/30, A61F 2/44

(54) **RHOMBOID SHAPED IMPLANTS**
RHOMBOIDFÖRMIGE IMPLANTATE
IMPLANTS DE FORME RHOMBOÏDE

(30) Priority: 27.05.2021 US 202117332284
(43) Date of publication of application: 10.04.2024
(73) Proprietor: Warsaw Orthopedic, Inc., Warsaw, IN 46582 (US)
(72) Inventor: DEWEY, Jonathan, Minneapolis, Minnesota 55432 (US); RUTH, Joshua Aaron, Minneapolis, Minnesota 55432 (US); PREVOST, Julien, Minneapolis, Minnesota 55432 (US)
(74) Representative: Viering, Jentschura & Partner mbB Patent- und Rechtsanwälte
(86) International application number: PCT/US2022/030094
(87) International publication number: WO 2022/251043

(56) References cited:
- EP-A1- 3 069 694
- WO-A1-2006/116850
- WO-A1-2015/063721
- WO-A1-2015/198335
- WO-A2-2012/139022
- CN-A- 107 137 166
- US-A1- 2010 152 853
- US-A1- 2019 328 539

## Description

### FIELD

The present technology is generally related to spinal implants for use in a medical procedure related to the spine. The implants may have a rhomboid like shape and may be used in an anterior cervical discectomy and fusion (ACDF) procedure although other uses in other areas of the spine and other orthopedic uses are also contemplated.

### BACKGROUND

Implants for the spine may be positioned between adjacent vertebrae of a patient include a superior endplate and an inferior endplate roughly having a rectangular and/or square configuration. Additionally, implants for the spine may include bone screw apertures extending through the superior endplate and/or inferior endplate for securing a corresponding bone screw into an adjacent vertebra.

From e.g. WO 2015 / 063 721 A1 a spinal implant is known, comprising a body extending from a proximal surface to a distal surface in a proximal-to-distal direction, extending from a first lateral surface to a second lateral surface in a widthwise direction, and extending from a superior surface to an inferior surface in a vertical direction, wherein: the proximal surface extends from a first lower end thereof to a first upper end thereof a first distance, the distal surface extends from a second lower end thereof to a second upper end thereof a second distance, the superior surface extends from the first upper end of the proximal surface to the second upper end of the distal surface a third distance, the third distance being defined between a central endpoint of the first upper end of the proximal surface to a central endpoint of the second upper end of the distal surface, the inferior surface extends from the first lower end of the proximal surface to the second lower end of the distal surface a fourth distance, the fourth distance being defined by a central endpoint of the first lower end of the proximal surface to a central endpoint of the second lower end of the distal surface, the first distance is greater than the second distance, and the third distance is less than the fourth distance.

Further spinal implants are known from e.g. WO 2015 / 198 335 A1, WO 2006 / 116 850 A1 and CN 107 137 166 A.

### SUMMARY

The techniques of this disclosure generally relate to an implant for the spine having a rhomboid like shape. A rhomboid or near-rhomboid shaped interbody as disclosed herein may more closely conform to the natural human anatomy of a disc space than conventional rectangular implants, particularly in the cervical area of the spine. Additionally, in various embodiments, the implant may, for example, orient a superior bone screw at an angle that is substantially or more closely approximately perpendicular with respect to a plane of the superior endplate. Similarly, the implant may, for example, orient an inferior bone screw at an angle that is substantially or more closely approximately perpendicular with respect to a plane of the inferior endplate.

That is, the present invention provides a spinal implant with the features according to claim 1. Further preferred embodiments of the implant are described in the dependent claims.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of an implant.
FIG. 2A is a first side view of the implant of FIG. 1.
FIG. 2B is a second side view of the implant of FIG. 1.
FIG. 2C is a third side view of the implant of FIG. 1.
FIG. 2D is a fourth side view of the implant of FIG. 1.
FIG. 3 is an alternate perspective view of an implant.
FIG. 4 is a perspective view of an alternate implant.
FIG. 5 is an alternate perspective view of the implant of FIG. 4.
FIG. 6A is a side view of a unibody implant in a contracted configuration.
FIG. 6B is a side view of the implant of FIG. 6A in an expanded configuration.

### DETAILED DESCRIPTION

Embodiments of the present disclosure relate generally, for example, to spinal interbody implants, and more particularly, to interbody implants that have a rhomboid like shape. Embodiments of the devices are described below with reference to the Figures.

The following discussion omits or only briefly describes certain components, features and functionality related to medical implants, installation tools, and associated surgical techniques, which are apparent to those of ordinary skill in the art. It is noted that various embodiments are described in detail with reference to the drawings, in which like reference numerals represent like parts and assemblies throughout the several views, where possible. Reference to various embodiments does not limit the scope of the claims appended hereto because the embodiments are examples of the inventive concepts described herein.

Terms such as "same," "equal," "planar," "coplanar," "parallel," "perpendicular," etc. as used herein are intended to encompass a meaning of exactly the same while also including variations that may occur, for example, due to manufacturing processes. The term "substantially" may be used herein to emphasize this meaning, particularly when the described embodiment has the same or nearly the same functionality or characteristic, unless the context or other statements clearly indicate otherwise.

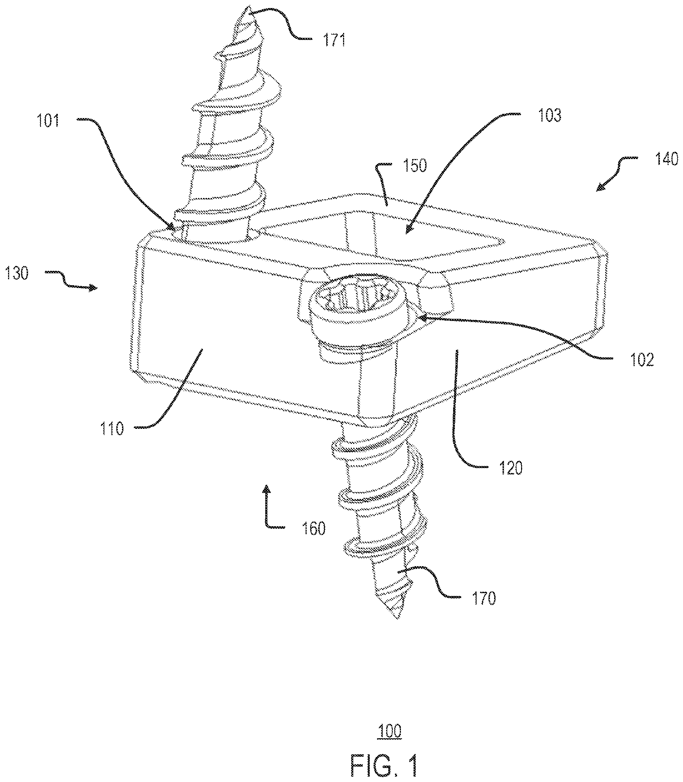

Referring generally to FIGS. 1-3 a first embodiment of a spinal implant 100 is disclosed. Spinal implant 100 may have a general shape approximating the shape of a rhomboid, for example. In a side view, example implants 100 may be shaped like a parallelogram in which adjacent sides are of unequal lengths and interior angles between the adjacent sides are non-right angled, i.e., have an approximately rhomboid shaped configuration. Example implants 100 may be of the expandable type or the non-expandable type. Those that do expand may form a general shape approximating the shape of a rhomboid in the expanded configuration, at least in cross section. Various surfaces of the implant 100 may be planar and/or concave, convex, undulating, etc. At least one advantage of implant 100 having a cross sectional shape that generally approximates a rhomboid is that implant 100 may better or more closely conform to and/or approximate a disc space of a patient, for example. For example, the cervical area of the spine may include disc spaces that are shaped like a trapezoid and a rhombus shaped implant may better support the disc space than a conventional square or rectangular shaped implant. Disclosed implants facilitate the maximization of endplate coverage within the disc space without protruding anteriorly, posteriorly, and/or into the spinal canal, for example.

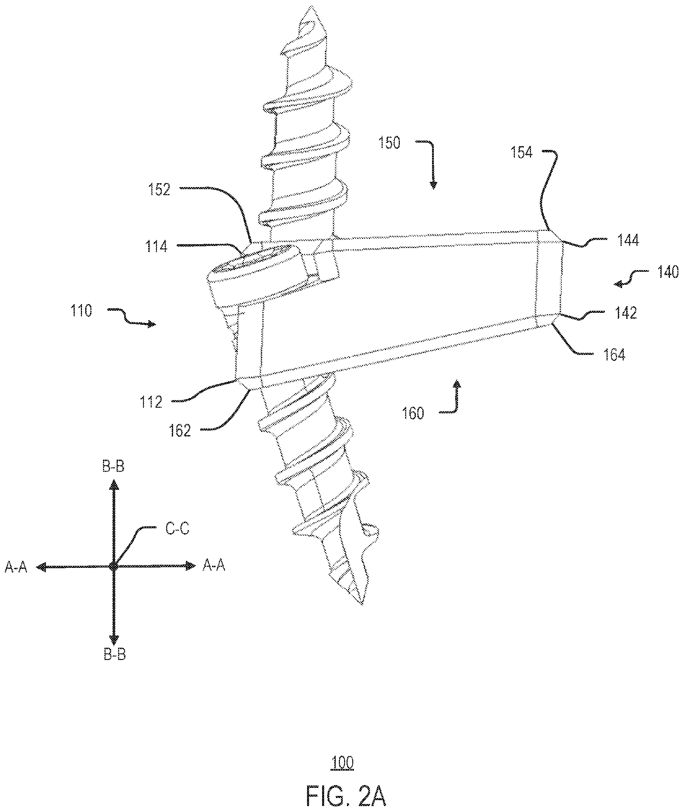

Implant 100 may be a unibody implant formed of a monolithic structure, or body, for example. Implant 100 may include a body having six dominant surfaces or sides, for example. Implant 100 may include proximal surface 110, first lateral surface 120, second lateral surface 130, distal surface 140, superior surface 150, and inferior surface 160. In some embodiments, superior surface 150 may be a surface of a superior endplate and inferior surface 160 may be a surface of an inferior endplate. The body of implant 100 may extend from proximal surface 110 to distal surface 140 in a proximal-to-distal direction A-A, extend from first lateral surface 120 to a second lateral surface 130 in a widthwise direction C-C, and extend from a superior surface 150 to an inferior surface 160 in a vertical direction B-B, for example (see FIG. 2A). In the example embodiment of FIG. 1, it is shown that a first bone screw aperture 101 extends through the superior surface 150 and a second bone screw aperture 102 extends through the inferior surface 160.

Referring to FIG. 2A, the proximal surface 110 may extend from a first lower end 112 to a first upper end 114, and the distal surface 140 may extend from a second lower end 142 to a second upper end 144, for example. The superior surface 150 may extend from a first proximal end 152 thereof, to a first distal end 154 thereof, and the inferior surface 160 may extend from a second proximal end 162 thereof to a second distal end 164 thereof, for example. In various embodiments, the edges of implant 100 may be chamfered, angled, rounded, and/or smooth. For example, the edge of implant 100 where proximal surface 110 meets the superior surface 150 may comprise a chamfered edge including both first upper end 114 of proximal surface 110 and end 152 of superior surface 150, for example. Similarly, the edge of implant 100 where distal surface 140 meets the superior surface 150 may comprise a chamfered edge including both second upper end 144 of distal surface 140 and end 154 of superior surface 150, for example. Likewise, the edge of implant 100 where proximal surface 110 meets inferior surface 160 may comprise a chamfered edge including both first lower end 112 of proximal surface 110 and end 162 of inferior surface 160, for example. Similarly, the edge of implant 100 where distal surface 140 meets the inferior surface 160 may comprise a chamfered edge including both second lower end 142 of distal surface 140 and end 164 of inferior surface 160, for example. In other embodiments, a chamfered edge may not be provided, or may be selectively provided, and the various edges may be the same, substantially the same, and/or similar as described above. Furthermore, in some embodiments, various surfaces described above having edges adjoining the chamfered edge may be coextensive.

In some embodiments, a transition from one surface to the next adjoining surface may not include a hard edge delimiting the two surfaces. For example, a transition from a dominant face of one surface to the next adjoining dominant face of another surface may be a smooth and relatively subtle transition. The smooth and/or relatively subtle transition may be a chamfered edge, a curved edge, a bulbous edge, or the like, for example. In some embodiments, the superior surface 150 may be convex and the curvature of the superior surface 150 may match the curvature of the endplate of an adjacent vertebra and the transition from the superior surface 150 to the first lateral surface 120 and second lateral surface 130 may be a smoother and/or gentler transition than what is shown in FIG.2A.

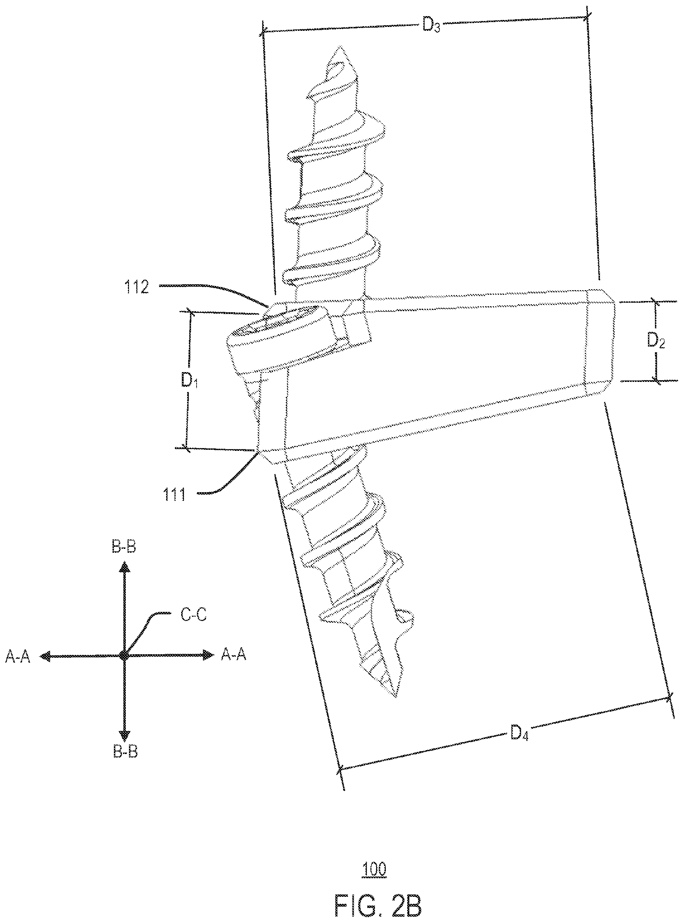

In FIG. 2B, a spinal implant 100 is illustrated. FIG. 2B is the same side view of implant 100 as FIG. 2A although with different geometrical properties emphasized. For example, a first distance D₁ of proximal surface 110 may be understood as a distance from first lower end 112 to first upper end 114 in the vertical direction B-B. For example, a first point on first lower end 112 that is laterally aligned in the widthwise direction C-C with a second point on first upper end 114, i.e., a corresponding point. A second distance D₂ of distal surface 140 may be understood as a distance from second lower end 142 to second upper end 144 in the vertical direction B-B. For example, a third point on second lower end 142 that is laterally aligned in the widthwise direction C-C with a fourth point on second upper end 144.

Similarly, a third distance D₃ of superior surface 150 may be understood as a distance from end 152 to end 154 (and/or also from first upper end 114 to second upper end 144) in the proximal-to-distal direction A-A. For example, a fifth point on end 152 that is laterally aligned in the widthwise direction C-C with a sixth point on first upper end 114, i.e., a corresponding point. A fourth distance D₄ of inferior surface 160 may be understood as a distance from end 162 to end 164. For example, a seventh point on end 162 that is laterally aligned in the widthwise direction C-C with an eighth point on end 164. In various embodiments, the fourth distance D₄ is greater than the first distance D₁, second distance D₂, and third distance D₃, for example. Additionally, the third distance may be greater than the first distance D₁ and the second distance D₂. Furthermore, the first distance D₁ may be greater than the second distance D₂. This relationship of distances may result in an implant 100 having a rhomboid and/or substantially rhomboid like shape. For example, in the side view of FIG. 2B the implant 100 is shaped like a rhomboid.

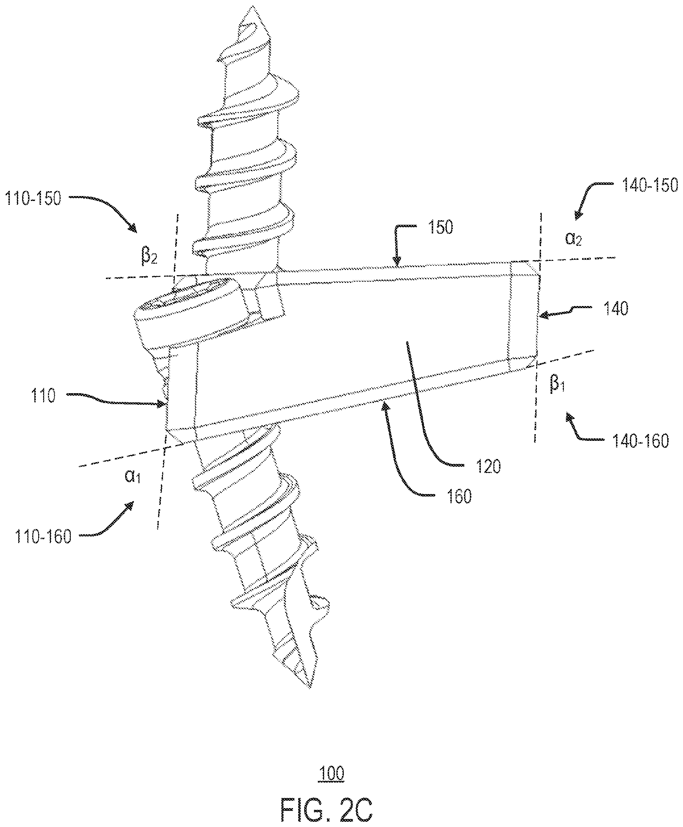

In various embodiments, the proximal surface 110, first lateral surface 120, second lateral surface 130, distal surface 140, superior surface 150, and inferior surface 160 may be substantially planar. In some embodiments, the proximal surface 110, first lateral surface 120, second lateral surface 130, distal surface 140, superior surface 150, and inferior surface 160 may include a portion thereof that may be substantially planar. For example, an end portion thereof and/or a central medial portion thereof. As illustrated in FIG. 2C, an extension of the dominant contour (and/or plane) of inferior surface 160 may intersect with an extension of the dominant contour of proximal surface 110 at point 110-160 and form a first acute angle α₁, i.e., an angle less than 90 degrees. Similarly, an extension of the dominant contour of inferior surface 160 may intersect with an extension of the dominant contour of distal surface 140 at point 140-160 and form a first obtuse angle β₁, i.e., an angle greater than 90 degrees and less than 180 degrees. Additionally, an extension of the dominant contour of superior surface 150 may intersect with an extension of the dominant contour of proximal surface 110 at point 110-150 and form a second obtuse angle β₂. Furthermore, an extension of the dominant contour of superior surface 150 may intersect with an extension of the dominant contour of distal surface 140 at point 140-150 and form a second acute angle α₂. In various embodiments, the above angles may be established at any point where the respective planes as explained above interest. Although an outside angle is labeled, those with skill in the art will appreciate that the labeled outside angle directly corresponds to the interior angle as well. Additionally, in some embodiments, the above angles are established at a point or points corresponding in space to the lateral ends adjacent the first lateral surface 120, and/or alternatively adjacent the second lateral surface 130, for example. Furthermore, the above angles may be established at a junction of the corresponding surfaces and correspond to an internal angle between the respective surfaces in the same, similar, and/or substantially the same way.

Consistent with the above disclosure, those with skill in the art will understand that the overall shape of implant 100 may be a generally rhomboid like shape when viewed from the side, and/or in a side view of a cross section through implant 100 in the proximal-to-distal direction and that the particular angles shown in the example embodiments may be different. For example, an exact angle between the edge portion of the superior surface 150 and the edge portion of the proximal surface 110 is not necessarily "obtuse" but rather the dominant contour of the superior surface 150 and the dominant contour of the proximal surface 110 intersect in 3D space to form an obtuse angle. Similarly, an exact angle between the edge portion of the inferior surface 160 and the proximal surface 110 is not necessarily "acute" but rather the dominant contour of the inferior surface 160 and the dominant contour of the proximal surface 110 intersect in 3D space to form an acute angle. In some embodiments, a dominant contour of a particular surface may be calculated as an average of various discrete contours of the same surface. For example, a dominant contour of the superior surface 150 may be calculated by taking an average of ten contour lines extending in a parallel direction from the proximal side of the superior surface 150 to the distal side of the superior surface 150, for example. Furthermore, a similar edge-to-edge averaging of contours of a different surface may be performed in like manner on any of the various surfaces discussed herein with more or less contour lines, e.g., any number of contour lines from about 2 to about 100 depending solely on the precision required for the target position of implant 100 between two particular vertebrae of a patient.

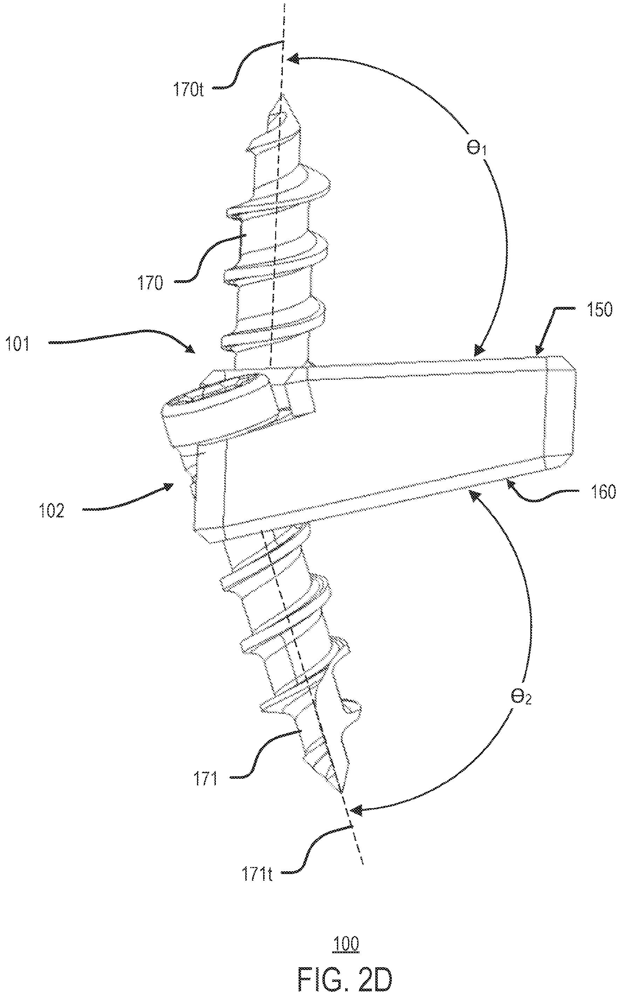

In the example embodiment of FIG. 2D, it is shown that a first bone screw aperture 101 extends through the superior surface 150 in a superior direction that is substantially parallel to the vertical direction, for example. Similarly, it is shown that a second bone screw aperture 102 extends through the inferior surface 160 in an inferior direction that is substantially parallel to the vertical direction. First bone screw aperture 101 may be formed at a corner of implant 100 formed partly in the proximal surface 110 and first lateral surface 120, for example. First bone screw aperture 101 may comprise a circular through hole, a tapering through hole, or a conical through hole that orients a first bone screw 170 in a pre-stablished trajectory 170t, for example. In various embodiments, trajectory 170t may project away from the superior surface 150 at an angle Θ₁ that is approximately and/or substantially perpendicular to superior surface 150, e.g., about 80-90 degrees. It is contemplated that trajectory 170t may be angled slightly in the distal direction and/or towards a medial portion of implant 100 as well. Similarly, second bone screw aperture 102 may comprise a circular through hole, a tapering through hole, or a conical through hole that orients a second bone screw 171 in a pre-stablished trajectory 171t, for example. In various embodiments, trajectory 171t may project away from the inferior surface 160 at an angle Θ₂ that is approximately and/or substantially perpendicular to inferior surface 160, e.g., about 80-90 degrees. It is contemplated that trajectory 171t may be angled slightly in the distal direction and/or towards a medial portion of implant 100 as well. In this way, trajectories 170t, 171t may converge towards a central portion of implant 100 from the proximal side of implant 100 towards the distal side of implant 100, for example. At least one advantage of bone screw apertures 101, 102 defining trajectories 170t, 171t as explained above is that the substantially perpendicular orientation increases the relative amount of shear loading the bone screws 170, 171 can absorb across the disc space compared to an axial force applied to the bone screws 170, 171, for example. Additionally, in various embodiments, the substantially or approximately perpendicular orientation facilitates the bone screws 170, 171 being secured to patient anatomy predominantly, mostly, and/or entirely within the anterior rim and/or cortical bone, which is generally considered a relatively strong section of bone.

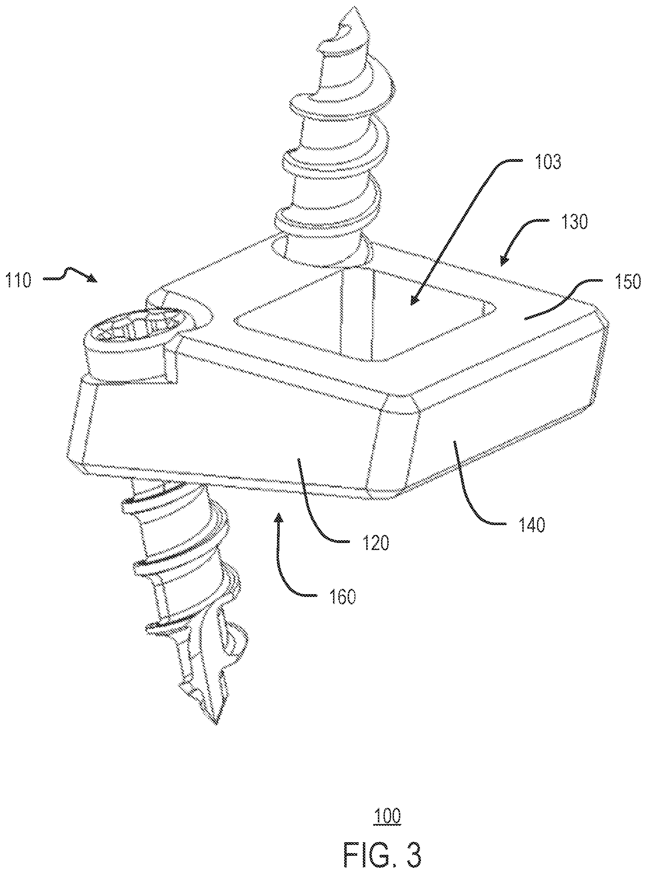

FIG. 3 is an additional perspective view of implant 100 showing the features and characteristics explained above. Additionally, FIG. 3 illustrates a bone graft aperture 103 that may be formed in the center of implant 100, for example in a medial position. Bone graft aperture 103 may extend through the superior surface 150 and inferior surface 160 and be shaped like a square, rectangle, oval, circle, or the like.

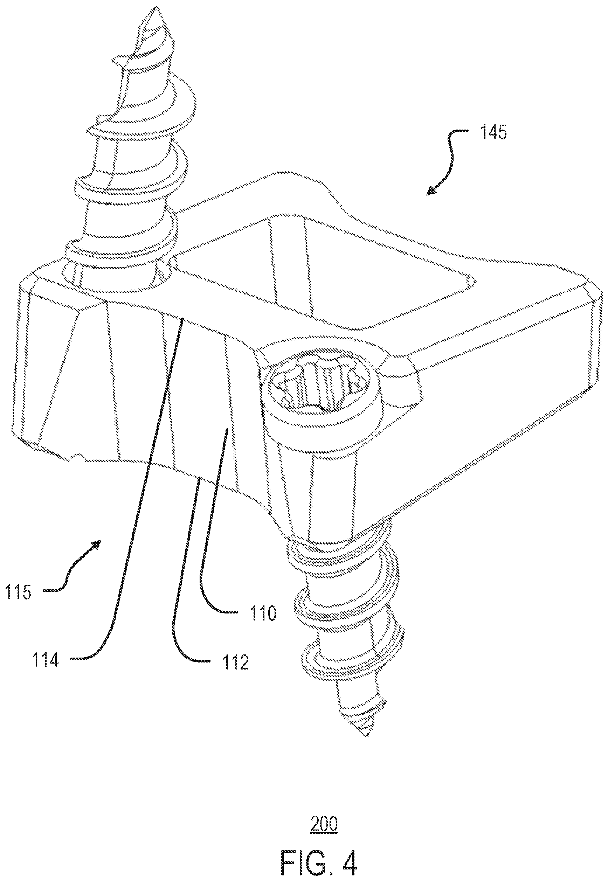

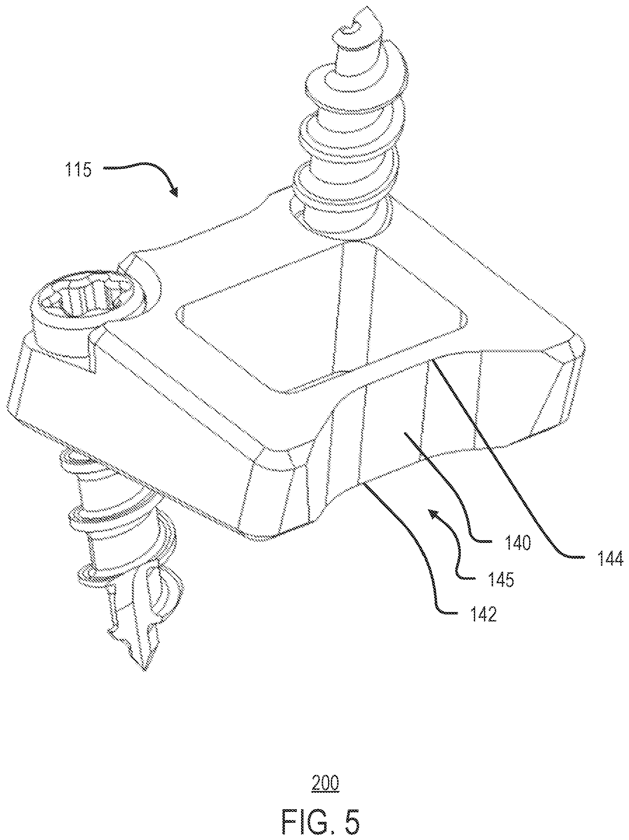

FIGS. 4-5 are perspective views of an alternate implant 200. Alternate implant 200 may have the same, similar, and/or substantially the same characteristics as explained above with respect to implant 100. Additionally, proximal surface 110 may include a relatively wide curved surface 115 (also referred to as a scallop 115), for example. In various embodiments, the proximal surface 110 may be substantially defined by a first curved surface 115 that arcs inward with respect to the distal surface 140 and extends in a vertical direction from the first lower end 112 to the first upper end 114, for example. Similarly, in various embodiments, distal surface 140 may include a relatively wide curved surface 145 (also referred to as a scallop 145), for example. In various embodiments, the distal surface 140 may be substantially defined by a second curved surface 145 that arcs inward with respect to the proximal surface 110 and extends in a vertical direction B-B from the first lower end 112 to the first upper end 114, for example. At least one advantage of curved surfaces 115, 145 is that they may be curved to avoid delicate anatomy such as the spinal cord and/or other sensitive patient tissue.

An additional advantage of curved surfaces 115, 145 is that they may accommodate and/or conform to additional medical hardware, such as an anterior plate for bone screws or the like (not illustrated). For example, curved surface 115 may allow access to install and/or tighten the bone screws to the adjacent vertebrae with a retaining plate in place. For example still, the curved surface 115 may allow the anterior plate to be positioned above the anterior face of the patient vertebrae a first distance and allow eyelets to be positioned proud by a second distance. In some embodiments, the first and second distances may be substantially the same which may allow the bone screws to be oriented perpendicular and/or substantially perpendicular to the patient VB which may allow the bone screws to be secured to cortical bone because the anterior plate has positioned the bone screws far enough in an anterior direction, for example.

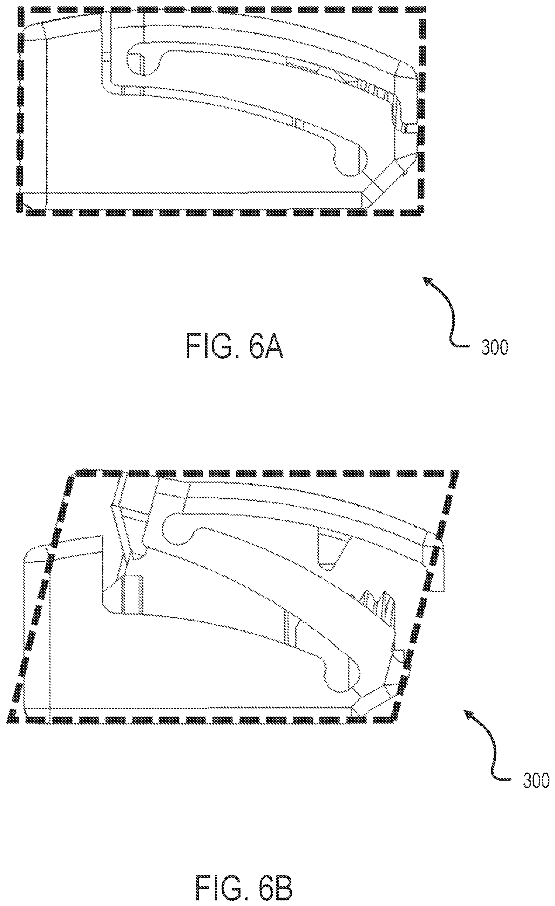

FIG. 6A is a side view of a unibody implant 300 in a contracted configuration and FIG. 6B is a side view of the unibody implant 300 in an expanded configuration. Unibody implant 300 may include the same, substantially the same, and/or similar features and characteristics as described in detail in U.S. App. No. 17/246,968, titled Unibody Dual Expanding Interbody Implant. Additionally, as illustrated, unibody implant 300 approximates a rhomboid like shape (in cross section). In some embodiments, unibody implant 300 may approximate a rhomboid like shape in an expanded position, a collapsed position, and/or an intermediate position. Accordingly, various unibody implants 300 may have the same advantages as disclosed herein with respect to implants 100, and 200. Similarly, the various implants disclosed in U.S. App. No. 17/307,578, titled Externally Driven Expandable Interbody and Related Methods; and U.S. App. No. 17/331,058, titled Dual Wedge Implant may also have a rhomboid like shape.

For example, features, functionality, and components from one embodiment may be combined with another embodiment and vice versa unless the context clearly indicates otherwise. Similarly, features, functionality, and components may be omitted unless the context clearly indicates otherwise.

Unless otherwise specifically defined herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an" and "the" include plural referents unless otherwise specified, and that the terms "comprises" and/ or "comprising," when used in this specification, specify the presence of stated features, elements, and/or components, but do not preclude the presence or addition of one or more other features, steps, operations, elements, components, and/or groups thereof.

## Claims

1. A spinal implant (100), comprising:
a body extending from a proximal surface (110) to a distal surface (140) in a proximal-to-distal direction, extending from a first lateral surface (120) to a second lateral surface (130) in a widthwise direction, and extending from a superior surface (150) to an inferior surface (160) in a vertical direction, and
a first bone screw aperture (101) defining a first trajectory (170t) for a first bone screw (170) and/or a second bone screw aperture (102) defining a second trajectory (171t) for a second bone (171) screw,
wherein:
the proximal surface (110) extends from a first lower end (112) thereof to a first upper end (114) thereof a first distance,
the distal surface (140) extends from a second lower end (142) thereof to a second upper end (144) thereof a second distance,
the superior surface (150) extends from the first upper (114) end of the proximal surface (110) to the second upper end (144) of the distal surface (140) a third distance, the third distance being defined between a central endpoint of the first upper (114) end of the proximal surface (110) to a central endpoint of the second upper end (144) of the distal surface (140),
the inferior surface (160) extends from the first lower end (112) of the proximal surface (110) to the second lower end (142) of the distal surface (140) a fourth distance, the fourth distance being defined by a central endpoint of the first lower end (112) of the proximal surface (110) to a central endpoint of the second lower end (142) of the distal surface (140),
the first distance is greater than the second distance, and
the third distance is less than the fourth distance, and
wherein:
the superior surface (150) is substantially planar and the first trajectory (170t) extends away from the superior surface (150) in a superior direction that is substantially perpendicular to the superior surface (150), and/or
the inferior surface (160) is substantially planar and the second trajectory (171t) extends away from the inferior surface (160) in an inferior direction that is substantially perpendicular to the inferior surface (160).

2. The spinal implant (100) of claim 1, wherein the fourth distance is greater than the first distance, the second distance, and the third distance.

3. The spinal implant (100) of claim 2, wherein the third distance is greater than the second distance and first distance.

4. The spinal implant (100) of claim 1, wherein, in a side view, the body is in a substantially rhomboid configuration.

5. The spinal implant (100) of claim 1, wherein the body is a unitary body.

6. The spinal implant (100) of claim 1, wherein the body is an expandable body and in an expanded configuration the body is in a substantially rhomboid configuration.

7. The spinal implant (100) of claim 1, wherein a centrally disposed graft aperture (103) extends through the superior surface (150) and the inferior surface (160) in the vertical direction.

8. The spinal implant (100) of claim 1, wherein:
a first angle at a first junction of the first upper end (114) of the proximal surface (110) and a proximal end of the superior surface (150) is greater than 90 degrees,
a second angle at a second junction of the second upper end (144) of the distal surface (140) and a distal end of the superior surface (150) is less than 90 degrees,
a third angle at a third junction of the first lower end (112) of the proximal surface (110) and a proximal end of the inferior surface (160) is less than 90 degrees, and
a fourth angle at a fourth junction of the second lower end (142) of the distal surface (140) and the inferior surface (160) is greater than 90 degrees.

9. The spinal implant (100) of claim 8, wherein each of the first through fourth junctions is chamfered.

10. The spinal implant (100) of claim 1, wherein:
the proximal surface (110) is substantially planar and defines a proximal plane,
the distal surface (140) is substantially planar and defines a distal plane,
the superior surface (150) is substantially planar and defines a superior plane, and
the inferior surface (160) is substantially planar and defines an inferior plane.

11. The spinal implant (100) of claim 10, wherein:
a first intersection of the proximal plane and the superior plane comprises a first interior angle that is greater than 90 degrees,
a second intersection of the distal plane and the superior plane comprises a second interior angle that is less than 90 degrees,
a third intersection of the proximal plane and the inferior plane comprises a third interior angle that is less than 90 degrees, and
a fourth intersection of the distal plane and the inferior plane comprises a fourth interior angle that is greater than 90 degrees.

12. The spinal implant (100) of claim 11, wherein:
a first perimeter of the proximal surface (110) is chamfered,
a second perimeter of the distal surface (140) is chamfered,
a third perimeter of the superior surface (150) is chamfered, and
a fourth perimeter of the inferior surface (160) is chamfered.

13. The spinal implant (100) of claim 1, wherein:
the proximal surface (110) is substantially defined by a first curved surface that arcs inward with respect to the distal surface (140) and extends in the vertical direction from the first lower end (112) of the proximal surface (110) to the first upper end (114) of the proximal surface (110), and
the distal surface (140) is substantially defined by a second curved surface that arcs inward with respect to the proximal surface (110) and extends in the vertical direction from the second lower end (142) of the distal surface (140) to the second upper end (144) of the distal surface (140).

## Patentansprüche

1. Wirbelsäulenimplantat (100), umfassend:
einen Körper, der sich von einer proximalen Oberfläche (110) zu einer distalen Oberfläche (140) in einer proximal-distalen Richtung erstreckt, sich von einer ersten seitlichen Oberfläche (120) zu einer zweiten seitlichen Oberfläche (130) in einer Breitenrichtung erstreckt und sich von einer superioren Oberfläche (150) zu einer inferioren Oberfläche (160) in einer vertikalen Richtung erstreckt, und
eine erste Knochenschraubenöffnung (101), die eine erste Trajektorie (170t) für eine erste Knochenschraube (170) definiert, und/oder eine zweite Knochenschraubenöffnung (102), die eine zweite Trajektorie (171t) für eine zweite Knochenschraube (171) definiert,
wobei:
die proximale Oberfläche (110) sich von einem ersten unteren Ende (112) davon zu einem ersten oberen Ende (114) davon über einen ersten Abstand erstreckt,
die distale Oberfläche (140) sich von einem zweiten unteren Ende (142) davon zu einem zweiten oberen Ende (144) davon über einen zweiten Abstand erstreckt,
die superiore Oberfläche (150) sich von dem ersten oberen (114) Ende der proximalen Oberfläche (110) zu dem zweiten oberen Ende (144) der distalen Oberfläche (140) über einen dritten Abstand erstreckt, wobei der dritte Abstand zwischen einem zentralen Endpunkt des ersten oberen (114) Endes der proximalen Oberfläche (110) zu einem zentralen Endpunkt des zweiten oberen Endes (144) der distalen Oberfläche (140) definiert ist,
die inferiore Oberfläche (160) sich von dem ersten unteren Ende (112) der proximalen Oberfläche (110) zu dem zweiten unteren Ende (142) der distalen Oberfläche (140) über einen vierten Abstand erstreckt, wobei der vierte Abstand durch einen zentralen Endpunkt des ersten unteren Endes (112) der proximalen Oberfläche (110) zu einem zentralen Endpunkt des zweiten unteren Endes (142) der distalen Oberfläche (140) definiert ist,
der erste Abstand größer als der zweite Abstand ist und
der dritte Abstand kleiner als der vierte Abstand ist und
wobei:
die superiore Oberfläche (150) im Wesentlichen eben ist und die erste Trajektorie (170t) sich von der superioren Oberfläche (150) weg in eine superiore Richtung erstreckt, die im Wesentlichen senkrecht zu der superioren Oberfläche (150) ist, und/oder
die inferiore Oberfläche (160) im Wesentlichen eben ist und die zweite Trajektorie (171t) sich von der inferioren Oberfläche (160) weg in eine inferiore Richtung erstreckt, die im Wesentlichen senkrecht zu der inferioren Oberfläche (160) ist.

2. Wirbelsäulenimplantat (100) nach Anspruch 1, wobei die vierte Abstand größer als der erste Abstand, der zweite Abstand und der dritte Abstand ist.

3. Wirbelsäulenimplantat (100) nach Anspruch 2, wobei der dritte Abstand größer als der zweite Abstand und der erste Abstand ist.

4. Wirbelsäulenimplantat (100) nach Anspruch 1, wobei, in einer Seitenansicht, der Körper in einer im Wesentlichen rhomboiden Konfiguration ist.

5. Wirbelsäulenimplantat (100) nach Anspruch 1, wobei der Körper ein einheitlicher Körper ist.

6. Wirbelsäulenimplantat (100) nach Anspruch 1, wobei der Körper ein expandierbarer Körper ist und in einer expandierten Konfiguration der Körper in einer im Wesentlichen rhomboiden Konfiguration ist.

7. Wirbelsäulenimplantat (100) nach Anspruch 1, wobei sich eine zentral angeordnete Transplantatöffnung (103) in der vertikalen Richtung durch die superiore Oberfläche (150) und die inferiore Oberfläche (160) erstreckt.

8. Wirbelsäulenimplantat (100) nach Anspruch 1, wobei:
ein erster Winkel an einer ersten Verbindungsstelle des ersten oberen Endes (114) der proximalen Oberfläche (110) und eines proximalen Endes der superioren Oberfläche (150) größer als 90 Grad ist,
ein zweiter Winkel an einer zweiten Verbindungsstelle des zweiten oberen Endes (144) der distalen Oberfläche (140) und eines distalen Endes der superioren Oberfläche (150) kleiner als 90 Grad ist,
ein dritter Winkel an einer dritten Verbindungsstelle des ersten unteren Endes (112) der proximalen Oberfläche (110) und eines proximalen Endes der inferioren Oberfläche (160) weniger als 90 Grad ist, und
ein vierter Winkel an einer vierten Verbindungsstelle des zweiten unteren Endes (142) der distalen Oberfläche (140) und der inferioren Oberfläche (160) größer als 90 Grad ist.

9. Wirbelsäulenimplantat (100) nach Anspruch 8, wobei jede der ersten bis einschließlich vierten Verbindungsstellen abgeschrägt ist.

10. Wirbelsäulenimplantat (100) nach Anspruch 1, wobei:
die proximale Oberfläche (110) im Wesentlichen eben ist und eine proximale Ebene definiert,
die distale Oberfläche (140) im Wesentlichen eben ist und eine distale Ebene definiert,
die superiore Oberfläche (150) im Wesentlichen eben ist und eine superiore Ebene definiert, und
die inferiore Oberfläche (160) im Wesentlichen eben ist und eine inferiore Ebene definiert.

11. Wirbelsäulenimplantat (100) nach Anspruch 10, wobei:
ein erster Schnittpunkt der proximalen Ebene und der superioren Ebene einen ersten Innenwinkel umfasst, der größer als 90 Grad ist,
ein zweiter Schnittpunkt der distalen Ebene und der superioren Ebene einen zweiten Innenwinkel umfasst, der kleiner als 90 Grad ist,
ein dritter Schnittpunkt der proximalen Ebene und der inferioren Ebene einen dritten Innenwinkel umfasst, der kleiner als 90 Grad ist, und
ein vierter Schnittpunkt der distalen Ebene und der inferioren Ebene einen vierten Innenwinkel umfasst, der größer als 90 Grad ist.

12. Wirbelsäulenimplantat (100) nach Anspruch 11, wobei:
ein erster Umfang der proximalen Oberfläche (110) abgeschrägt ist,
ein zweiter Umfang der distalen Oberfläche (140) abgeschrägt ist,
ein dritter Umfang der superioren Oberfläche (150) abgeschrägt ist, und
ein vierter Umfang der inferioren Oberfläche (160) abgeschrägt ist.

13. Wirbelsäulenimplantat (100) nach Anspruch 1, wobei:
die proximale Oberfläche (110) im Wesentlichen durch eine erste gekrümmte Oberfläche definiert ist, die hinsichtlich der distalen Oberfläche (140) nach innen gebogen ist und sich in der vertikalen Richtung von dem ersten unteren Ende (112) der proximalen Oberfläche (110) zu dem ersten oberen Ende (114) der proximalen Oberfläche (110) erstreckt, und
die distale Oberfläche (140) im Wesentlichen durch eine zweite gekrümmte Oberfläche definiert ist, die hinsichtlich der proximalen Oberfläche (110) nach innen gebogen ist und sich in der vertikalen Richtung von dem zweiten unteren Ende (142) der distalen Oberfläche (140) zu dem zweiten oberen Ende (144) der distalen Oberfläche (140) erstreckt.

## Revendications

1. Implant rachidien (100) comprenant :
un corps s'étendant d'une surface proximale (110) à une surface distale (140) dans un sens distal à proximal, s'étendant d'une première surface latérale (120) à une seconde surface latérale (130) dans un sens de la largeur, et s'étendant d'une surface supérieure (150) à une surface inférieure (160) dans un sens vertical, et
une première ouverture de vis à os (101) définissant une première trajectoire (170t) pour une première vis à os (170) et/ou une seconde ouverture de vis à os (102) définissant une seconde trajectoire (171t) pour une seconde vis à os (171),
dans lequel :
la surface proximale (110) s'étend d'une première extrémité inférieure (112) de celui-ci à une première extrémité supérieure (114) de celui-ci sur une première distance,
la surface distale (140) s'étend d'une seconde extrémité inférieure (142) de celui-ci à une seconde extrémité supérieure (144) de celui-ci sur une deuxième distance,
la surface supérieure (150) s'étend de la première extrémité supérieure (114) de la surface proximale (110) à la seconde extrémité supérieure (144) de la surface distale (140) sur une troisième distance, la troisième distance étant définie entre un point d'extrémité central de la première extrémité supérieure (114) de la surface proximale (110) et un point d'extrémité central de la seconde extrémité supérieure (144) de la surface distale (140),
la surface inférieure (160) s'étend de la première extrémité inférieure (112) de la surface proximale (110) à la seconde extrémité inférieure (142) de la surface distale (140) sur une quatrième distance, la quatrième distance étant définie par un point d'extrémité central de la première extrémité inférieure (112) de la surface proximale (110) et un point d'extrémité central de la seconde extrémité inférieure (142) de la surface distale (140),
la première distance est supérieure à la deuxième distance, et
la troisième distance est inférieure à la quatrième distance, et
dans lequel :
la surface supérieure (150) est sensiblement plane et la première trajectoire (170t) s'étend à l'écart de la surface supérieure (150) dans un sens supérieur qui est sensiblement perpendiculaire à la surface supérieure (150), et/ou
la surface inférieure (160) est sensiblement plane et la seconde trajectoire (171t) s'étend à l'écart de la surface inférieure (160) dans un sens inférieur qui est sensiblement perpendiculaire à la surface inférieure (160).

2. Implant rachidien (100) selon la revendication 1, dans lequel la quatrième distance est supérieure à la première distance, à la deuxième distance et à la troisième distance.

3. Implant rachidien (100) selon la revendication 2, dans lequel la troisième distance est supérieure à la deuxième distance et à la première distance.

4. Implant rachidien (100) selon la revendication 1, dans lequel, en vue latérale, le corps est dans une configuration sensiblement rhomboïdale.

5. Implant rachidien (100) selon la revendication 1, dans lequel le corps est un corps unitaire.

6. Implant rachidien (100) selon la revendication 1, dans lequel le corps est un corps expansible et, dans une configuration expansée, le corps est dans une configuration sensiblement rhomboïde.

7. Implant rachidien (100) selon la revendication 1, dans lequel une ouverture de greffe (103) disposée au centre s'étend à travers la surface supérieure (150) et la surface inférieure (160) dans le sens vertical.

8. Implant rachidien (100) selon la revendication 1, dans lequel :
un premier angle à une première jonction de la première extrémité supérieure (114) de la surface proximale (110) et une extrémité proximale de la surface supérieure (150) est supérieur à 90 degrés,
un deuxième angle à une deuxième jonction de la seconde extrémité supérieure (144) de la surface distale (140) et une extrémité distale de la surface supérieure (150) est inférieur à 90 degrés,
un troisième angle à une troisième jonction de la première extrémité inférieure (112) de la surface proximale (110) et une extrémité proximale de la surface inférieure (160) est inférieur à 90 degrés, et
un quatrième angle à une quatrième jonction de la seconde extrémité inférieure (142) de la surface distale (140) et de la surface inférieure (160) est supérieur à 90 degrés.

9. Implant rachidien (100) selon la revendication 8, dans lequel chacune des jonctions de la première à la quatrième est chanfreinée.

10. Implant rachidien (100) selon la revendication 1, dans lequel :
la surface proximale (110) est sensiblement plane et définit un plan proximal,
la surface distale (140) est sensiblement plane et définit un plan distal,
la surface supérieure (150) est sensiblement plane et définit un plan supérieur, et
la surface inférieure (160) est sensiblement plane et définit un plan inférieur.

11. Implant rachidien (100) selon la revendication 10, dans lequel :
une première intersection du plan proximal et du plan supérieur comprend un premier angle intérieur supérieur à 90 degrés,
une deuxième intersection du plan distal et du plan supérieur comprend un deuxième angle intérieur inférieur à 90 degrés,
une troisième intersection du plan proximal et du plan inférieur comprend un troisième angle intérieur inférieur à 90 degrés, et
une quatrième intersection du plan distal et du plan inférieur comprend un quatrième angle intérieur supérieur à 90 degrés.

12. Implant rachidien (100) selon la revendication 11, dans lequel :
un premier périmètre de la surface proximale (110) est chanfreiné,
un deuxième périmètre de la surface distale (140) est chanfreiné,
un troisième périmètre de la surface supérieure (150) est chanfreiné, et
un quatrième périmètre de la surface inférieure (160) est chanfreiné.

13. Implant rachidien (100) selon la revendication 1, dans lequel :
la surface proximale (110) est sensiblement définie par une première surface incurvée qui forme un arc vers l'intérieur par rapport à la surface distale (140) et s'étend dans le sens vertical de la première extrémité inférieure (112) de la surface proximale (110) à la première extrémité supérieure (114) de la surface proximale (110), et
la surface distale (140) est sensiblement définie par une seconde surface incurvée qui forme un arc vers l'intérieur par rapport à la surface proximale (110) et s'étend dans le sens vertical de la seconde extrémité inférieure (142) de la surface distale (140) à la seconde extrémité supérieure (144) de la surface distale (140).
